(19) Europäisches Patentamt / European Patent Office / Office européen des brevets

(11) **EP 2 268 362 B1**

(12) **EUROPEAN PATENT SPECIFICATION**

(45) Date of publication and mention
of the grant of the patent:
**01.03.2017 Bulletin 2017/09**

(21) Application number: **09730385.3**

(22) Date of filing: **07.04.2009**

(51) Int Cl.:
*A61Q 19/00* (2006.01)     *A61Q 19/10* (2006.01)
*A61K 8/02* (2006.01)     *A61K 8/06* (2006.01)
*A61K 8/19* (2006.01)     *A61K 8/73* (2006.01)
*C11D 17/04* (2006.01)

(86) International application number:
**PCT/IL2009/000398**

(87) International publication number:
**WO 2009/125405 (15.10.2009 Gazette 2009/42)**

(54) **USE OF POLYSACCHARIDE POLYMERS AND CATIONS FOR THE PREPARATION OF WET WIPES**

VERWENDUNG VON POLYSACCHARID-POLYMEREN UND KATIONEN ZUR HERSTELLUNG VON FEUCHTTÜCHERN

UTILISATION DE POLYMÈRES DE POLYSACCHARIDES ET DE CATIONS POUR LA PRÉPARATION DE LINGETTES HUMIDES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **10.04.2008 GB 0806537**

(43) Date of publication of application:
**05.01.2011 Bulletin 2011/01**

(73) Proprietor: **Albaad Massuot Yitzhak Ltd.**
**79858 Massuot Yitzhak (IL)**

(72) Inventors:
• **HAMMER, Ifat**
**76302 Rehovot (IL)**

• **KUPERMINTZ, Miriam**
**65787 Tel Aviv (IL)**

(74) Representative: **Colombo, Stefano Paolo et al**
**Marchi & Partners S.r.l.**
**Via Vittor Pisani, 13**
**20124 Milano (IT)**

(56) References cited:
**WO-A-01/78678          WO-A-02/49604**
**WO-A-99/13820          WO-A-2006/053333**
**WO-A-2007/144819      WO-A-2008/007059**
**US-A1- 2003 027 473   US-A1- 2003 113 364**
**US-A1- 2005 158 369   US-A1- 2007 238 634**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

This invention relates to the field of wet wipes.

BACKGROUND OF THE INVENTION

Disposable wet wipes and disposable towelettes pre-moistened by impregnation with a liquid are well known in the art and are used for personal care of babies, children and adults.

Impregnated wet wipes typically consist of a dry substrate, usually a non-woven fabric which may be produced using several different technologies of laying and bonding, and a low-viscosity liquid which may be water dispersion or an emulsion, typically an oil in water emulsion. Non-woven fabric is typically prepared by a manufacturing process comprising two basic manufacturing steps, the first one being laying of the fibers and forming a web and the second being a bonding stage. Laying technologies are known in the art, e.g. wet-laying, air-laying or carding. Bonding technologies are also known in the art, e.g. latex bonding, thermo-bonding fibers or hydro-entanglement by water jets. Often, the non-woven fabric is carded and bonded by hydro-entanglement, a technology known as Spunlace.

The non-woven fabric is typically converted, cut, impregnated with liquid, folded, stacked in layers and packed as wet wipes either in soft wraps (such as flow-wrapped laminated films) or in different types of boxes.

The liquid is typically a composition containing water (80% or more) and combinations of various other ingredients including humectants, emollients, surfactants, emulsifiers, anti-microbial agents, perfumes, pH adjusting agents, active ingredients.

The current converting technology requires low viscosity liquids of about 50 mPas and no more than 2000 mPas, most preferably 50-250 mPas, as the liquid is sprayed onto the non-woven fabric through nozzles. Due to this spraying step only low viscous impregnation liquids can be used resulting in limited possibilities for the design of impregnation liquids, limited selection of water insoluble ingredients, limited stability of the impregnation liquids and further results in the need for sophisticated emulsifying systems.

The known wipe products provide for skin cleansing and deliver ingredients beneficial for the skin to the skin of the user. However, known wet wipe products do not provide a cosmetic, pleasant, esthetic and sensorial perception to the user, similar to cosmetic creams, rich lotions, gels and lotions available in jars, bottles or other packaging forms. For example, subjects using known wet wipes do not experience a thick, soft, creamy and silky feel when applying the wet wipe to the skin enabling a rich lotion, gel or cream, derived from the wet wipe, to be spread on the skin easily during application similar to a cosmetic cream from e.g. a jar.

SUMMARY OF THE INVENTION

It is thus an object of the invention to provide thixotropic liquid compositions which can be impregnated into wet wipes thereby enabling the delivery of viscous liquids to the skin by "wet wipe delivery".

The subject invention thus provides a novel wet wipe which upon use by the consumer deliver a highly viscous cream (or paste or gel or lotion) to the skin which can be spread on the skin similar to known cosmetic creams available in e.g. jars and bottles. The subject invention further provides a method for producing the novel wet wipe of the invention and uses thereof.

Thus, in one aspect of the present invention, there is provided a wet wipe product, as defined in claim 1.

The invention further provides a method of producing a wet wipe product, as defined in claim 4.

BRIEF DESCRIPTION OF THE DRAWINGS

In order to understand the invention and to see how it may be carried out in practice, embodiments will now be described, by way of non-limiting example only, with reference to the accompanying drawings, in which:

Figure 1 shows D-mannuronic acid and L-guluronic acid, i.e. the algin monomers.
Figure 2 shows block shapes in alginates.
Figure 3 shows the "egg box" configuration of the alginate polymer which occurs when the polymer is contacted with a divalent cation, i.e. calcium.
Figure 4 shows the thixotropic behavior of a sodium alginate based lotion of the invention.

DETAILED DESCRIPTION OF THE INVENTION

It is an object of the invention to provide thixotropic liquid compositions which can be impregnated into wet wipes thereby enabling the delivery of viscous liquids to the skin via the wet wipe.

The term "*thixotropic*" as used herein should be understood to encompass the rheological behavior of a pseudoplastic fluid having flow (viscosity) which is time-dependent. At constant applied shear rate, the viscosity of a thixotropic fluid gradually decreases.

The present invention provides a use of a liquid composition in combination with a substrate to form a wet wipe product which upon topical application to the skin is capable of affecting cleansing and cosmetic treatment of the skin. The wet wipe product of the invention provides the consumer with a rich cream, paste, gel or lotion which can be spread on the skin and deliver cosmetic benefits and silky, soft sensorial properties to the skin of the consumer.

Thus, in one aspect of the present invention, there is provided a wet wipe product, as defined in claim 1.

As used herein the term "*liquid composition*" should be understood to encompass a liquid, a solution, an aqueous dispersion, a gel, an emulsion, a water-in-oil emulsion, an oil-in-water emulsion, a paste, a lotion.

Compositions as used herein may comprise:

- water, a polysaccharide polymer and cations; or
- water and a polysaccharide polymer; or
- water and cations.

As used herein, the term "*enriched with a polysaccharide polymer*" should be understood to encompass enriched with a composition comprising a polysaccharide polymer. Similarly, as used herein, the term "*enriched with a cation*", e.g. a calcium cation, should be understood to encompass enriched with a composition comprising a cation.

As used herein, the term "*substrate*" should be understood to encompass a material or fabric which is non-woven, woven, or porous material which is capable of holding the composition within the pores of the substrate.

As used herein, the term "*impregnate*" should be understood to encompass the filling (saturating), partial or complete, of voids and interstices in a material or substrate with a compound or a composition. For example, impregnation may be carried out by spraying a liquid on non-woven fabric (f.e. through nozzles).

As used herein, the term "*enriched*" should be understood to encompass adding or supplementing, independent of the manner of addition or supplementation. For instance, enrichment of fabric with cations, such as calcium ions, is carried out by impregnation or by soaking or by other methods known in the art of non-woven manufacturing.

As used herein, the term "*non-woven*" fabric should be understood to encompass any non-woven material or fabric. Non-limiting examples of non-woven material/fabric are materials/fabrics based on cellulose fibers, biopolymers such as, but not limited to polylactic acid, synthetic polymeric fibers such as, but not limited to polyester, polypropylene and combinations thereof.

Thus, in one embodiment of the invention, the substrate is a non-woven fabric. In a further embodiment the non-woven substrate comprises fibers selected from the group consisting of cellulose fibers, polyester fibers, polypropylene fibers, polylactic acid fibers and mixtures thereof.

Non-woven materials may include one or more layers of fibrous material, e.g. a laminate of fibrous material. The separate layers may be formed of similar or dissimilar materials.

In one embodiment, a non-woven fabric substrate may contain from 1% to 100% by weight cellulose fibers and from 100% to 1% by weight synthetic polymeric fibers or biopolymers.

The term "*wipe product*" or "*wet wipe product*" or "*wet wipe*" as used herein should be understood to encompass a combination of a substrate as used in the invention and a (liquid) composition of the present invention, pre-combined for later use. The term "*disposable (wet) wipe (product)"* as used herein should be understood to encompass wipe products which are intended to be discarded after a single use, i.e. the wipe product in its whole is not intended to be laundered or otherwise restored or reused as a wipe product, even though certain materials or all of the wipe product may be recycled, reused, or composted.

Compositions and wipe products of the invention are useful in skin cleaning, skin cleansing and cosmetic treatment of the skin.

As used herein the term "*cream*" should be understood to encompass, a cream, a gel, a paste, a (rich) lotion.

As used herein, the term "*upon use*" as used herein should be understood to encompass the moment from which the subject applies a wet wipe of the invention to the skin.

When preparing a wipe product according to the invention, i.e. a wet wipe comprising a cream, a liquid composition is prepared which has low viscosity when freshly made. The viscosity of this liquid composition increases after from 3 hours to 24 hours and becomes a high viscous cream as a result of a reaction between a polysaccharide polymer (present in the liquid composition) and cations (present in the liquid composition). In one embodiment, the cations are present in the liquid composition. In this embodiment, the reaction may occur in the liquid composition prior to, during and/or after impregnation. In yet another embodiment, the cations are both present in the liquid composition and in the substrate. In this embodiment, the reaction may occur in the liquid composition prior to, during and/or after impregnation and also in or on the substrate impregnated with the liquid composition. The reaction is independent of the location of the cations which may either be in the liquid composition or alternatively in the substrate.

As used herein, the term "*high viscous*" or "*high viscosity*" should be understood to encompass at least 1000 mPas. Preferably above 2000 mPas.

As used herein, the term "*low viscous*" or "*low viscosity*" should be understood to encompass at least 50 mPas and not more than 1000 mPas, preferably 50-250 mPas.

As used herein, the term "*cations*" should be understood to encompass any atom or molecule which has lost at least one valence electron, making it positively charged. Non-limiting examples of such cations are calcium cations, magnesium cations, potassium cations, sodium cations, molybdenum cations, zinc cations, copper cations, manganese cations, strontium cations, barium cations, iron cations, aluminum cations, bismuth cations.

The term "*monovalent cations*" as used herein should be understood to encompass any atom or molecule which has lost one valence electron, making it charged with a +1 charge. Non-limiting examples of such monovalent cations are potassium cations ($K^+$), sodium cations ($Na^+$) and molybdenum cations ($Mb^+$),

The term "*bivalent cations*" as used herein should be understood to encompass any atom or molecule which has lost two valence electrons, making it charged with a +2 charge. Non-limiting examples of such bivalent cations are calcium cations, ($Ca^{+2}$), magnesium cations, ($Mg^{+2}$), zinc cations ($Zn^{+2}$), copper cations ($Cu^{+2}$), manganese cations ($Mn^{+2}$), nickel cations ($Ni^{+2}$), strontium cations ($Sr^{+2}$), barium cations ($Ba^{+2}$), iron cations ($Fe^{+2}$), aluminium cations ($Al^{+2}$), bismuth cations, ($Bi^{+2}$) and so forth.

The cations are bivalent cations and are calcium cations.

Non-limiting examples of calcium salts (source of calcium cations) are calcium chloride, calcium phosphate, calcium stearate, calcium sulfate, calcium carbonate, calcium acetate, calcium ascorbate, calcium aspartate, calcium benzoate, calcium citrate, calcium gluconate, calcium lactate, calcium pantothenoate, calcium paraben, calcium PCA, calcium propionate, calcium saccharin, calcium sorbate, and calcium tartarate.

The calcium cations are employed in a concentration of from 0.01 to 0.03 weight%.

A (liquid) composition of the subject description is based on one or more polysaccharide polymers such as, but not limited to alginates, alginate salts, carrageenan polysaccharides (derived from red seaweeds), pectins, biosacharide gum-1, biosacharide gum-2, biosacharide gum-3, biosacharide gum-4. Non-limiting examples of alginate salts are sodium alginates, potassium alginates, calcium alginates, ammonium alginates, propylene glycol alginates and so forth.

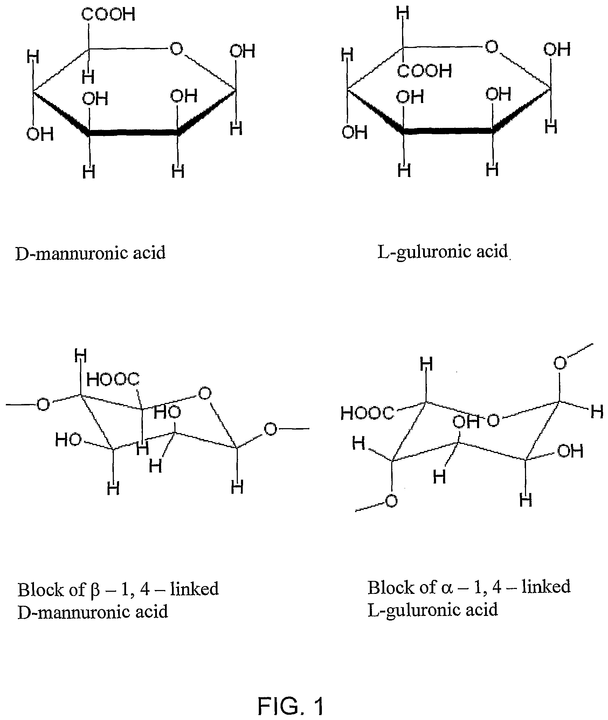

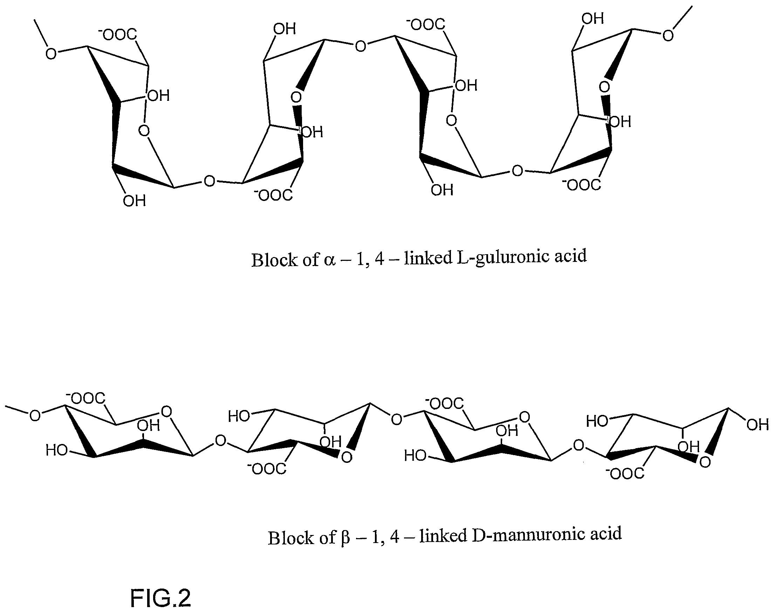

An "*alginate*" is a linear co-polymer composed of two monomeric units: D-mannuronic acid and L-guluronic acid. These monomers occur in the alginate molecule as regions made up exclusively of one unit or the other, referred to as M-blocks or G-blocks, or as regions in which the monomers approximate an alternating sequence. The shapes of the individual monomers are shown in Figure 1. The D-mannuronic acid exists in the 1C conformation and is connected in the alginate polymer in the β-configuration through the 1- and 4- positions. The L-guluronic acid has the 1C conformation and is α-1,4-linked in the polymer. Because of the particular shapes of the monomers and their 01889625\18-01 modes of linkage in the polymer, the geometries of the G-block regions, M-block regions, and alternating regions are substantially different as shown in Figure 2.

Without being bound by theory, the gelation mechanism of polysaccharides by which thixotropic material may be formed, may include the formation of ionic bonds between polymeric chains and cations. It should be understood that the term "*ionic bond*" encompasses a chemical bond that is formed through electrostatic attraction.

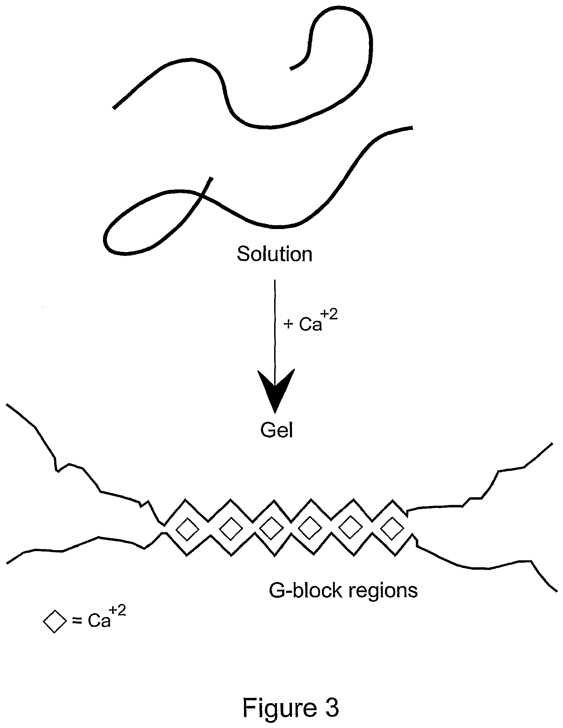

Without being bound by theory, it is pointed out that, the calcium reactivity of alginates is a consequence of the particular molecular geometries of each of these regions. When bivalent cations such as calcium cations are added to a sodium alginate solution, a special alignment of the G-blocks occurs and the calcium cations are bound between the two chains. This conformation of the polymer can be visualized as "eggs in an egg box" like conformation, as shown in Figure 3.

Without being bound by theory, the calcium reactivity of alginates is the result of calcium-induced dimeric association of the G-block regions. Depending on the amount of calcium present in the system, these inter-chain associations can be either temporary or permanent. When using a low level of calcium, temporary associations are obtained, giving rise to high viscous thixotropic solutions. The thixotropic properties enable flow when high shear forces are applied.

In one embodiment of the invention, the polysaccharide polymer is selected from a group consisting of an alginate, an alginate salt, or mixtures thereof. In one embodiment, the alginate salt is sodium alginate. In a further embodiment of the invention, the alginate or alginate salt contains calcium cations.

In one embodiment of the invention, the polysaccharide polymer is sodium alginate and the cations are calcium cations.

In one embodiment, an alginate is employed in an amount of from 0.05 to 1.0 weight %. In another embodiment, an alginate is employed in an amount of from 0.1 to 0.6 weight %. In yet another embodiment, an alginate is employed in an amount of from 0.2 to 0.4 weight %.

In another aspect of the invention there is provided a method of producing a wet wipe product, as defined in claim 4.

An example of a composition of the present invention is an emulsion comprising water, a lipid (oily) phase, an emulsifier, an alginate polymer and a calcium salt.

In one embodiment of the present invention the composition comprised in the wet wipe product is an oil-in-water emulsion. In yet another embodiment the emulsion comprises from 0.5% to 20% w/w of an oil, a fat or a lipid. In another embodiment the dry weight of the emulsion is from about 2 to about 5 times the dry weight of the substrate.

In an embodiment of the present invention, a wet-wipe product of the invention further comprises at least one agent selected from the group consisting of an anti-microbial agent, a pH-adjusting agent, a chelating agent, a water-soluble polyol, a perfume ingredient, a powder, a humectant (moistener), a skin soothing aid, a plant extract, a cosmetic active ingredient, an emollient, a fragrance and mixtures thereof.

Non-limiting examples of plant-extracts are Aloe Barbadensis, Chamomilla Recutita Extract, Achillea Millefolium Extract, Algae Extract, Calendula Officinalis Extract, Cucumis Sativus (Cucumber) Extract, Hippophae Rhamnoides Extract, Lavandula Angustifolia (Lavender) Extract, Rosa Canina Extract, Zingiber Aromaticus Extract, Camelia Sinensis (Greentea) Extract and so forth.

Non-limiting examples of powders are Zinc Oxide, Potato Starch, Corn Starch, Silica, Bentonite, Montmorillonite, Hectorite, Kaolin, Polyethylene, Talc and so forth.

Non-limiting examples of cosmetic active ingredients are Tocopheryl Acetate, Alanine, Arginine, Allantoin, Caffeine, Ceramide, Collagen, Niacinamide, Ubiquinone, Retinyl Palmitate, Sphingolipids, and so forth.

Non-limiting examples of skin soothing aids are acetyl cysteine, acetyl glutamine, alanine, ammonium caseinate, avena sativa, behenyl betaine, beta-glucan, bioflavanoids, bisabolol, capryloyl salicylic acid, carnitine, ceramide-1, ceramide-2, ceramide-3, ceramide-4, ceramide-5, cetearyl phosphate, glycyrrhizic acid, lactoferrin, mineral salts, squalene, xanthan gum and so forth.

Non-limiting examples of humectants are Sorbitol, Glycerin, Hexylene Glycol, Butylene Glycol, Pentylene Glycol, Ethylhexylglycerin, Panthenol, Glucose, Fructose, PEG-200, Urea, Caprylyl Glycol and so forth.

Water serves as a medium for carrying surfactants, emollients, humectants and so forth to the skin in an esthetically pleasant manner and at the requested high viscosity of a paste to a cream. In addition, water aids in wetting of the substrate of wipe products incorporating a liquid composition according to the invention. The water must be deionized in order to provide the exact and necessary amount of cations such as calcium cations. In one embodiment, a composition of the present invention comprises at least 70% water by weight of the composition. In another embodiment, a composition of the present invention comprises at least about 80% water by weight of the composition. In yet another embodiment, a composition of the present invention comprises up to about 98% water by weight of the composition. In a specific embodiment, a composition of the present invention comprises from about 85% to about 90% water by weight of the composition.

Emollients provide emolliency and moisturizing properties to the finished wet wipe product. In addition, emollients also lubricate the skin surface and tend to release soil such as dirt, grease and make-up residues, to thereby facilitate skin cleaning. Preferred oils are those that impart a tactile impression of softness and smoothness, and which do not impart an excessive tactile perception of greasiness, oiliness, or coating, when incorporated into the composition. Non-limiting examples of emollients are oils, fats and lipids such as, but not limited to White Mineral Oil (Paraffinum Liquidum), Ethylhexyl Palmitate, and Caprylic/Capric Triglycerides, Caprylic/Capric Triglyceride, Cyclomethicone, Dimethicone, C12-15 Alkyl Benzoate, Cetyl Esters, Palm Glycerides, Sucrose Oleate, Propylene Glycol Caprylate, Octadecane, Octyldodecanol, Oleyl Oleate, Myristyl Alcohol, Jojoba Esters, Ethylhexyl Isononanoate, 12-20 Isoparaffin and so forth.

In one embodiment, the emollients are employed in an amount of at least about 0.5% and up to about 15%, by weight of the composition. In a specific embodiment, the emollients are employed in an amount from about 0.5% to about less than 4%, based on the weight of the composition.

A composition of the invention may also include one or more emulsifiers known in the art of forming oil-in-water emulsions. Such emulsifiers are employed in an amount effective to emulsify the oils and other non-water-soluble oils that may be present in the composition. In one embodiment, an emulsifier is employed in an amount ranging from about 0.5% to about 10% based on the weight of the composition. In another embodiment, an emulsifier is employed in an amount ranging from about 1% to about 7% based on the weight of the composition. In yet another embodiment, mixtures of emulsifiers may be used. Non-limiting examples of emulsifiers which may be used are Polyglycerol esters, Sucrose esters, fatty Alcohols, Phosphate esters, Sorbitan esters and so forth.

In one embodiment, the composition may further contain one or more components selected from water-soluble polyols, pH-adjusting agents, anti-microbial agents and chelating agents. Such components may form a preservative system.

It is generally desired in personal cleansing or treating compositions to adjust the pH of the composition to that, or near that, of skin. Therefore, in one embodiment, the pH will be adjusted to from about 4 to about 7. In another embodiment, the pH will be adjusted to from about 4.5 to about 6.5. The pH can be adjusted by adding one or more pH-adjusting agents in an amount effective to provide such pH values. Non-limiting examples of pH adjusting agents are organic and inorganic acids and bases such as, but not limited to Acetic acid and salts, Citric Acid and salts, Ammonium Hydroxide, Sodium Hydroxide, Ethanolamine, Lactic Acid and salt, Malic acid and salts, Phosphoric Acid and salts and so forth.

The amount of the pH-adjusting agent that is employed depends on the equivalent weight of the pH-adjusting agent and the desired pH. In one embodiment, the pH-adjusting agent is used in an amount of from about 0.01 to about 0.5 weight % of the composition. In another embodiment, the pH-adjusting agent is used in an amount of from about 0.05 to about 0.2 weight % of the composition. In yet another embodiment, the pH-adjusting agent is used in an amount of from about 0.1 to about 0.2 weight % of the composition.

Anti-microbial agents (preservatives) may improve shelf life of a composition of the invention and a wipe product of the invention incorporating such composition. Non-limiting examples of anti-microbials as used in the invention are parabens and their salts, Methylisothiazolinone, Ethylhexylglycerin, Organic Acids and their salts such as, but not limited to, Sorbic Acid, Dehydroacetic Acid, Benzoic Acid and so forth.

An anti-microbial agent or preservative agent may be used in an amount effective to provide a suitable shelf life of the wet wipe product.

A "*(suitable) shelf life*" as used herein should be understood to encompass a shelf life resulting in storage stability for at least 24 months at room temperature, i.e., microorganisms do not grow to a significant extent during this time period.

In one embodiment, a product of the invention has a shelf life of at least 30 months under storage conditions of about 20-25°C

In one embodiment, the anti-microbial agent is methylisothiazolinone having a concentration in the range of between about 0.005w/w% to about 0.0095 w/w%; In another embodiment, the anti-microbial agent is phenoxyethanol having a concentration of between about 0.5w/w% to about 0.8 w/w%; In another embodiment, the anti-microbial agent is methylparaben having a concentration of between about 0.1w/w% to about 0.25w/w%; In another embodiment, the anti-microbial agent is Phenoxyethanol having a concentration of between about 0.5w/w% to about 1w/w%; In another embodiment, the anti-microbial agent is Dehydroacetic acid having a concentration of between about 0.03w/w% to about 0.06w/w%; In yet another embodiment, the anti-microbial agent is Benzoic Acid having a concentration of between about 0.03w/w% to about 0.06w/w%

In another embodiment the preservation system used is composed of methylisothiazolinone 0.005-0.0095w/w%, phenoxyethanol 0.5-0.8w/w% and methylparaben 0.1-0.25w/w%. In yet another embodiment the preservation system used is composed of 0.5-1w/w% Phenoxyethanol, 0.03-0.06w/w% Dehydroacetic acid and 0.03-0.06w/w% benzoic acid. In yet another embodiment the preservation system used is composed of 0.7-1w/w% Phenoxyethanol and Ethylhexylglycerin 0.8-1.5w/w%. In yet another embodiment the preservation system used is composed of 0.005-0.0095% methylisothiazolinone and 0.04-0.07% 2-bromo-2-nitropropane-1,3-diol.

In one embodiment, wherein the fabric is enriched with bivalent cations, e.g. enriched with calcium cations, the level of calcium in the fabric is 1 gram calcium (pure) per $1m^2$. In another embodiment, the level of calcium in the fabric is 0.2 -0.6 gram per $1 m^2$. In yet another embodiment, the level of calcium in the fabric is 0.3-0.5 gram for $1 m^2$.

Non-woven fabric used in the subject invention is manufactured according to methods known in the art. Typically, the basic steps include

a) Web Laying, which includes weighing the fibers, mixing them in desired concentrations, fiber opening and carding.
b) Bonding which includes entanglement of the fibers with water jets or other methods which are known in the art of non woven manufacturing. A stable network is formed which can optionally be subjected to embossment if a specific pattern is desired in the finished fabric.
c) Drying which includes the dewatering (sucking) of the fabric and drying; and
d) Conversion which includes cutting the fabric into the desired dimensions, impregnating with a liquid composition, folding, stacking in layers and packing as wet wipes either in soft wraps (such as flow-wrapped laminated films) or in different types of boxes.

In one embodiment, cations, e.g. bivalent cations, for example in the form of a calcium salt, are impregnated in the fabric by incorporation of the calcium salt into the water system which is used for the entanglement of the fibers.

In another embodiment, cations, e.g. bivalent cations, for example in the form of a calcium salt, are impregnated in the fabric through impregnation as a solution after the dewatering stage and before the drying step of the fabric.

In yet another embodiment, cations e.g. bivalent cations, for example in the form of a calcium salt, are impregnated in the fabric by dividing the drying step into two or more stages and impregnating the calcium salt before the final drying step.

In yet another embodiment, cations, e.g. bivalent cations, for example in the form of a solution of a calcium salt, are impregnated during converting, before or along with the liquid impregnation.

In one embodiment, further to the addition of cations (such as calcium cations) to the fabric, a sequestrant is added in order to delay the cation (e.g. calcium) release during mixing of the ingredients and avoid formation of a stiff gel before impregnation. Non-limiting examples of sequestrants that can be used in the subject invention are sodium salts such as, but not limited to, sodium hexametaphosphate, tetrasodium pyrophosphate, sodium citrate and so forth. A sequestrant competes with the cations, e.g. bivalent cations such as calcium cations, thereby decreasing the gelling rate.

A composition of the invention may be prepared by oil-in-water emulsion techniques known in the art. Typically, a process involves solubilizing e.g. an alginate in hot water, or, under high shear forces. Thereafter, a mixture of other ingredients of the composition is prepared and subjected to conditions to cause formation of a homogeneous and stable oil-in-water emulsion. A stable emulsion remains homogeneous over a given period which is determined by the required shelf life of the composition.

In one embodiment, such a mixture may be formed in one step by addition and mixing of each of the ingredients. In another embodiment, less than all of the ingredients may be pre-combined for subsequent combination with other ingredients or other pre-combined ingredients to form the mixture. For example, an antimicrobial agent may be pre-mixed with a portion of the water, water-soluble polyol, or a mixture thereof.

In a specific embodiment, e.g. the alginate and the calcium salt are solubilized in hot water. The oily phase with the emulsifiers is heated and mixed in separate containers. Thereafter the alginate phase and the oily phase are combined and homogenized under high shear forces. Equipment suitable for forming the mixtures and emulsion is known in the art.

To form the emulsion, in one embodiment, the mixture is mixed for a period of from about 5 to 60 minutes, with a motor speed set to drive a free shaft at a rate of from about 1,000 rpm to about 15,000 rpm. In another embodiment, the motor speed is set to drive a free shaft at a rate of from about 1000 rpm to about 8000 rpm.

In another embodiment, the mixture is mixed for a period of from about 5 to 30 minutes, with the motor speed set to drive a free shaft at a rate of from about 3000 rpm to about 5000 rpm.

As will be understood by the skilled artisan, care should be taken to avoid subjecting the mixture to excessive shear, which may interfere with emulsification. After homogenization the solution is cooled down while stirring.

Non-woven fabrics may be air-laid, wet-laid, spun-laid, melt blown, or carded. Non-woven materials may be treated, for example, to join fibers of the non-woven material or to enhance the strength of the non-woven material. Such treatment may involve hydro-entanglement, thermal bonding, or treatment with a binder.

Techniques for combining wipe substrates with a cleansing or treating composition and for their packaging are well known in the art and are applicable to the present invention.

In one embodiment, a wipe substrate is combined with a composition of the invention by spraying.

In one embodiment, wipe substrates as used in the invention are combined with a composition of the invention in an amount of from about 2 to about 5 times, preferably about 2.2 to about 4.2 times the dry weight of the substrate. For example, a wipe of the present invention may contain a composition of the invention in an amount of about 3 times the dry weight of the non-woven substrate.

The invention is further described in the following examples, which are not in any way intended to limit the scope of the inventions as claimed.

EXAMPLES

EXAMPLE 1 - Method for preparing a wet wipe by impregnation of an emulsion composition

Phase A

| | |
|---|---|
| Aqua | q.s |
| Calcium Chloride Solution 20% | 0.100% |
| Sodium Alginate | 0.330% |

Phase B

| | |
|---|---|
| Glyceryl Stearate | 3.700% |

(continued)

| | |
|---|---|
| Polyglyceryl-3 Stearate | 3.700% |
| Ethylhexyl Palmitate | 3.300% |
| Coco-Caprylate Caprate | 0.500% |

Phase C

| | |
|---|---|
| Preservatives | q.s |
| Tocopheryl Acetate | 0.500% |
| Parfum | q.s |
| pH adjustors | q.s |

RO water was heated to 80°C and Calcium Chloride and Sodium Alginate were added while homogenizing. The ingredients of phase B were mixed and heated to 80°C. Phase B was added to Phase A and homogenized. The emulsion was cooled to 40°C and phase C was added. The resulting solution was impregnated on the wipes. The impregnation was carried out by spraying the liquid on the non-woven fabric through nozzles.

EXAMPLE 2 - Method for preparing a gel composition

| | |
|---|---|
| Aqua | q.s |
| Calcium Chloride Solution 20% | 0.100% |
| Sodium Alginate | 0.400% |
| Potassium Sorbate | 0.200% |
| Methylisothiazolinone | 0.0095% |
| Citric Acid | q.s |

A preparation tank was filled with RO water. Calcium chloride was added and stirred for 5 minutes until fully dissolved. Alginate salt was added and homogenized under 5000 RPM for 5 minutes. The preservatives Methylisothiazolinone and Potassium Sorbate were added. The pH was adjusted. The impregnation was carried out by spraying the liquid on the non-woven fabric through nozzles.

EXAMPLE 3 - Method for preparing a wet wipe by impregnation with an alginate salt solution when the fabric is impregnated with calcium cations

I. Preparation of the liquid

Phase A

| | |
|---|---|
| Aqua | q.s |
| Sodium Alginate | 0.330% |

Phase B

| | |
|---|---|
| Glyceryl Stearate | 3.700% |
| Polyglyceryl-3 Stearate | 3.700% |
| Ethylhexyl Palmitate | 3.300% |

(continued)

| Dimethicone | 0.500% |
|---|---|

Phase C

| Preservatives | q.s |
|---|---|
| Tocopheryl Acetate | 0.500% |
| Parfum | q.s |
| pH adjustors | q.s |

RO water was heated to 80°C and Sodium Alginate was added while homogenizing for 10 minutes at 400 RPM. The ingredients of phase B were mixed and heated to 80°C. Phase B was added to Phase A and homogenized. The emulsion was cooled to 40°C and phase C was added. The resulting solution was impregnated on the wipes. The impregnation was carried out by spraying the liquid on the non-woven fabric through nozzles.

*II. Preparation of non-woven fabric with calcium level of 0.4 gr/m2*

50 gr/m2 Spunlace fabric having a Calcium Chloride content of 0.02 gr per 1 m2 was produced as follows:

The fabric was composed of Viscose and Polyester fibers in a ratio of 1:1. The fibers were weighed, mixed and passed a process of fine opening. The fibers were then carded and entangled by water jets. Thereafter the fabric was dewatered by sucking. After the sucking stage it was impregnated with a solution of Calcium Chloride. The concentration of the Calcium Chloride solution was calculated according to the following equation:

$$A = \frac{B * 100}{(C + D) * IR}$$

wherein:

A = Concentration of Calcium Chloride solution (%)
B = required quantity of Calcium Chloride per 1 $m^2$ in grams.
C = desired Weight of 1 $m^2$ dry fabric
D= Amount of water bound to 1 $m^{2\ after}$ sucking stage.
IR = Impregnation ratio of the Calcium Chloride solution (determined by convenience of facility)

In the subject example, B was 0.02 gr; C was 50 gr; D was 50 gr (depends on machinery and specific to non-woven plant facilities); and IR = 1
Concentration of Calcium Salt solution % is calculated as follows:

$$A = \frac{0.02 * 100}{(50 + 50) * 1} = 0.02\%$$

EXAMPLE 4 - Method for preparing a thixotropic lotion with Carrageenan

| Aqua | q.s |
|---|---|
| Potassium Sorbate | 0.3500% |
| Cargeenan (Blend E1, CP Kelco) | 0.500% |
| Premix emulsion (Emulgade CM, Cognis) | 0.200% |

(Cetearyl Alcohol, Glyceryl Stearate, Glycerin, Ceteareth-12, Cetyl Palmitate)

| | |
|---|---|
| Methylisothiazolinone | 0.0095% |
| Citric Acid | q.s |

A preparation tank was filled with RO water; Potassium Sorbate was added and stirred for 5 minutes until fully dissolved; Carrageenan was added and homogenized at 5000 RPM for 5 minutes; the preservative Methylisothiazolinone was added; and the pH was adjusted as requested.

EXAMPLE 5 - thixotropic behavior of a sodium alginate based lotion

The thixotropic behavior of the following sodium alginate based lotion composition was measured for 30 minutes

| | |
|---|---|
| Aqua | q.s |
| Calcium Chloride Solution 20% | 0.070% |
| Sodium Alginate | 0.330% |
| Glyceryl Stearate | 3.700% |
| Polyglyceryl-3 Stearate | 3.700% |
| Ethylhexyl Palmitate | 3.300% |
| Dimethicone | 0.500% |
| Phenoxyethanol | 0.432% |
| Methylparaben | 0.096% |
| Propylparaben | 0.048% |
| Ethylparaben | 0.024% |
| Parfum | 0.100% |

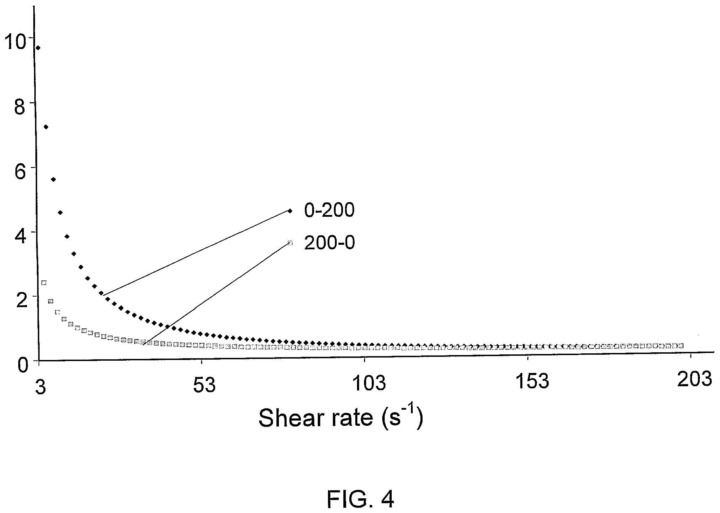

Figure 4 demonstrates the viscosity (Pas) as a function of shear rate (0-200 s$^{-1}$).

The measurements were made on a Thermo Haake RheoScope (Thermo Electron GmbH, Karlsruhe, Germany), using a cone (6 cm diameter, 1 grad. angle) and glass plate.

Figure 4 demonstrates that when the sample was exposed to increasing shear forces during 30 minutes, a definite shear thinning was noticed. After reducing the shear rate the viscosity was built back again. Considerable measurable delay in the viscosity recovery was detected. Without being bound by theory, the hysteresis may have been a result of a physical transformation (weakening of the materials) within the structures, which took place in the shear rates of 60-70 s$^{-1}$.

The viscosity of the alginate based thixotropic composition increased further when adsorbed on the fabric due to water binding of the fabric.

**Claims**

1. A wet wipe product consisting of a substrate and an oil-in-water emulsion composition comprising (i) at least 70%w/w water, (ii) a polysaccharide polymer being an alginate, an alginate salt, or mixtures thereof, (iii) from 0.01 to 0.03 w/w% calcium cation, and (iv) an oil, a fat or a lipid in an amount of from 0.5% to 20% w/w, the wet wipe product being **characterized in that** the substrate is impregnated with the composition and the composition becomes a high viscous cream having a viscosity of at least 1000mPas after from 3 hours to 24 hours, as a result of a ionic bonding reaction between said polysaccharide polymer and said cation.

2. The wet wipe product according to claim 1, wherein the alginate salt is sodium alginate.

3. A product according to any one of claims 1 to 2, wherein the composition comprises at least one agent selected from the group consisting of an anti-microbial agent, a pH-adjusting agent, a chelating agent, a water-soluble polyol, a perfume ingredient, a powder, a humectant, a skin soothing aid, a plant extract, a cosmetic active ingredient, an emollient, a fragrance and mixtures thereof.

4. A method for producing a wet wipe product according to any one of Claims 1 to 3 comprising the following step:

impregnating a substrate with an oil-in-water emulsion composition comprising water, an alginate, an alginate salt, or mixtures thereof, calcium cations, and an oil, a fat or a lipid.

**Patentansprüche**

1. Feuchttuchprodukt bestehend aus einem Trägermaterial und einer Öl-in-Wasser-Emulsionszusammensetzung mit

    - (i) wenigstens 70 Gew.-% Wasser,
    - (ii) einem Polysaccharid-Polymer, welches ein Alginat, ein Alginatsalz oder eine Mischung hieraus ist,
    - (iii) aus 0,01 bis 0,03 Gew.-% Kalziumkationen und
    - (iv) einem Öl, einem Fett oder einem Lipid in einer Menge von 0,5 bis 20 Gew.-%,

    wobei das Feuchttuchprodukt **dadurch gekennzeichnet ist,**
    **dass** das Trägermaterial mit der Zusammensetzung imprägniert ist und die Zusammensetzung nach 3 bis 24 Stunden als ein Ergebnis einer Ionenbindungsreaktion zwischen dem Polysaccharid-Polymer und den Kationen zu einer hochviskosen Creme mit einer Viskosität von zumindest 1000 mPas geworden ist.

2. Feuchttuchprodukt nach Anspruch 1,
    wobei das Alginatsalz Natriumalginat ist.

3. Erzeugnis nach einem der Ansprüche 1 bis 2,
    wobei die Zusammensetzung mindestens ein Mittel aufweist, welches ausgewählt ist aus der Gruppe bestehend aus einem antimikrobiellen Mittel, einem pHeinstellendem Mittel, einem Komplexbildner, einem wasserlöslichen Polyol, einem Duftstoff, einem Pulver, einem Befeuchtungsmittel, einem Hautpflegemittel, einem Pflanzenextrakt, einem kosmetisch aktivem Bestandteil, einem Weichmacher, einem Parfum und Mischungen hieraus.

4. Verfahren zum Herstellen eines Feuchttuchproduktes nach einem der Ansprüche 1 bis 3, mit den folgenden Schritten:

    Imprägnieren eines Trägermaterials mit einer Öl-in-Wasser-Emulsionszusammensetzung mit Wasser, einem Alginat, einem Alginatsalz oder Mischungen hiervon, Kalziumkationen und einem Öl, einem Fett oder einem Lipid.

**Revendications**

1. Produit de type lingette humide constitué d'un substrat et d'une composition d'émulsion huile dans l'eau comprenant (i) au moins 70 % en p/p d'eau, (ii) un polymère de polysaccharide étant un alginate, un sel d'alginate, ou un mélange de ceux-ci, (iii) de 0,01 à 0,03 % en p/p de cations de calcium et (iv) une huile, une graisse ou un lipide en une quantité de 0,5 % à 20 % en p/p, le produit de type lingette humide étant **caractérisé en ce que** le substrat est imprégné de la composition et **en ce que** la composition devient une crème hautement visqueuse ayant une viscosité d'au moins 1 000 mPas après 3 heures à 24 heures, suite à une réaction de liaison ionique entre ledit polymère de polysaccharide et ledit cation.

2. Produit de type lingette humide selon la revendication 1, dans lequel le sel d'alginate est l'alginate de sodium.

3. Produit selon l'une quelconque des revendications 1 à 2, dans lequel la composition comprend au moins un agent choisi dans le groupe constitué d'un agent antimicrobien, d'un agent d'ajustement du pH, d'un agent chélatant, d'un polyol soluble dans l'eau, d'un ingrédient parfumé, d'une poudre, d'un humectant, d'un auxiliaire d'adoucissement de la peau, d'un extrait végétal, d'un principe actif cosmétique, d'un émollient, d'une fragrance et de mélanges de ceux-ci.

4. Procédé de production d'un produit de type lingette humide selon l'une quelconque des revendications 1 à 3 comprenant l'étape suivante :

    imprégnation d'un substrat avec une composition d'émulsion huile dans l'eau comprenant de l'eau, un alginate, un sel d'alginate, ou un mélange de ceux-ci, des cations de calcium, et une huile, une graisse ou un lipide.

D-mannuronic acid

L-guluronic acid

Block of β – 1, 4 – linked
D-mannuronic acid

Block of α – 1, 4 – linked
L-guluronic acid

FIG. 1

Block of α − 1, 4 − linked L-guluronic acid

Block of β − 1, 4 − linked D-mannuronic acid

FIG.2

Solution

$+ \ Ca^{+2}$

Gel

G-block regions

$\lozenge = Ca^{+2}$

Figure 3

FIG. 4